# EUROPEAN PATENT SPECIFICATION

(11) **EP 2 869 853 B1**
(45) Date of publication and mention of the grant of the patent: **22.11.2017**
(21) Application number: 13762254.4
(22) Date of filing: 03.07.2013
(51) Int. Cl.: A61L 9/14

(54) **SYSTEM FOR THE EXECUTION, TRACEABILITY, MONITORING AND CONTROL OF A METHOD OF REDUCING THE BACTERIAL COUNT IN A CONFINED ENVIRONMENT**
SYSTEM ZUR AUSFÜHRUNG, VERFOLGBARKEIT, ÜBERWACHUNG UND STEUERUNG EINES VERFAHRENS ZUR REDUZIERUNG DER KEIMZAHL IN EINER GESCHLOSSENEN UMGEBUNG
SYSTÈME POUR L'EXÉCUTION, LA TRAÇABILITÉ, LA SURVEILLANCE ET LA COMMANDE D'UN PROCÉDÉ DE RÉDUCTION DE LA NUMÉRATION BACTÉRIENNE DANS UN ENVIRONNEMENT CONFINÉ

(30) Priority: 04.07.2012 IT TO20120589
(43) Date of publication of application: 13.05.2015
(73) Proprietor: WORKINPROGRESS ITALIA S.R.L., 20121 Milano (IT)
(72) Inventor: LONG, Matteo, 10010 Bairo (Torino) (IT)
(74) Representative: Fioravanti, Corrado
(86) International application number: PCT/IB2013/055449
(87) International publication number: WO 2014/006577

(56) References cited:
- EP-A1- 1 790 360
- WO-A1-2009/138430
- WO-A2-2012/033850
- US-A1- 2006 008 379

## Description

The present invention relates to a system for the execution, traceability, monitoring and control of a method of reducing the bacterial count in a confined environment, such as a laboratory or a clean room.

WO 2012/033 850 discloses a device for disinfecting a room, the device comprises a dry fogging disinfection module and a sensor for monitoring the sterilant concentration in the room.

WO 2009/138 430 discloses a device and method that involves determining value of a relative of the air in space, and atomizing a disinfecting liquid in the space until a predetermined value of the relative humidity of the air is reached in the space. The relative humidity of the air is maintained for a predetermined time at the latter value by atomizing the disinfecting liquid. The relative humidity of the air in the space is decreased before or during atomizing of the disinfecting liquid. The disinfecting liquid is atomized by passing the disinfecting liquid through a nozzle.

US 2006/008 379 discloses an apparatus and a method that involves introducing hydrogen peroxide in the form of a vapour to a room. A residual hydrogen peroxide is removed up to one concentration level after a period of time sufficient to decontaminate the room. A condensing-type dehumidifier is operated until the hydrogen peroxide concentration reaches another concentration level, after the hydrogen peroxide concentration has reached the former concentration level.

At the present time, the quantitative and qualitative bacterial level in a confined environment is maintained by the use of a number of predetermined manual operating procedures, such as cleaning and the removal of organic material present in the confined environment and on the surfaces located therein, the disinfection or sanitization of these surfaces by the application of decontaminant chemical products, and the rinsing and drying of said surfaces.

The monitoring of the operational activities of sanitization and disinfection of the aforesaid confined environments is therefore carried out manually by an operator, and is documented in a record or "self-monitoring plan" which may be accompanied by qualitative bacterial sampling conducted by qualified external operators on the air and surfaces of the confined environment.

It follows from the above that the monitoring activity cannot be validated scientifically and automatically, owing to the large number of manual operations that it requires; instead, the operator employed to perform these operations signs a simple documented self-certification of the activities performed.

This very commonly gives rise to errors of transcription, while also entailing a risk of bacterial exposure for the operator, with repercussions on the operator's health and on the planned activity within the confined environment.

Object of the present invention is therefore to propose a system for the execution, traceability, monitoring and control of a method of reducing the bacterial count in a confined environment which enables the risk of human error in the activities of disinfection and sanitization of the confined environment to be reduced or eliminated, in order to ensure the attainment of an expected quantitative and qualitative bacterial objective, while providing automatic and error-free traceability of each individual operation relating thereto and certifying the bacterial result obtained.

These and other objects are achieved by means of a system for the execution, traceability, monitoring and control of a method of reducing the bacterial count in a confined environment, the principal characteristics of which are defined in Claim 1.

Specific embodiments are described in the dependent claims, the content of which is to be considered as an integral and essential part of the present description.

Further characteristics and advantages of the present invention will be made clear by the following detailed description, provided purely by way of non-limiting example, with reference to the attached drawings, in which:
- Figure 1 is a perspective front view of the system according to the present invention, applied in a confined environment;
- Figure 2 is a front view of the environment identification sensor of the system according to the invention;
- Figure 3 is a front view of the device for micronizing decontaminant substances;
- Figure 4 is a front view of the detection sensor and the instantaneous bacterial analysis sensor.

Briefly, the system according to the invention can be used to enable and control, by acquiring all the technical and architectonic data of each individual confined environment for which bacterial quality maintenance is required, the activity of a device for micronizing decontaminant substances present in the confined environment.

The micronizing device distributes a decontaminant substance uniformly within the confined environment, without leaving residues on the surfaces, and calibrates its activity of bacterial count reduction (decontamination) in order to achieve an expected quantitative and qualitative bacterial objective.

This method of reducing the bacterial count in a confined environment is applied in conformity with the modes of use specified in the technical and safety data sheets for the decontaminant substances used and in the user's manuals of the micronizing device. The bacterial count reduction in a confined environment is carried out with respect to quantitative and qualitative parameters predetermined by the users, to allow the certification of the bacterial result obtained.

The system according to the invention identifies all the disinfection and sanitization operations carried out within the confined environment and the type of decontaminant substances used for the purpose of achieving the expected objective.

By using an instantaneous bacterial analysis sensor, activated before or at the end of the bacterial count reduction method, the system of the present invention is capable of carrying out immediate bacterial quantity and quality sampling and providing official certification of the result obtained.

The acquired information can be used for the creation of a database, the processing of statistical parameters and the analysis of the costs and other information relating to the activity of qualitative environmental maintenance, for each individual confined environment.

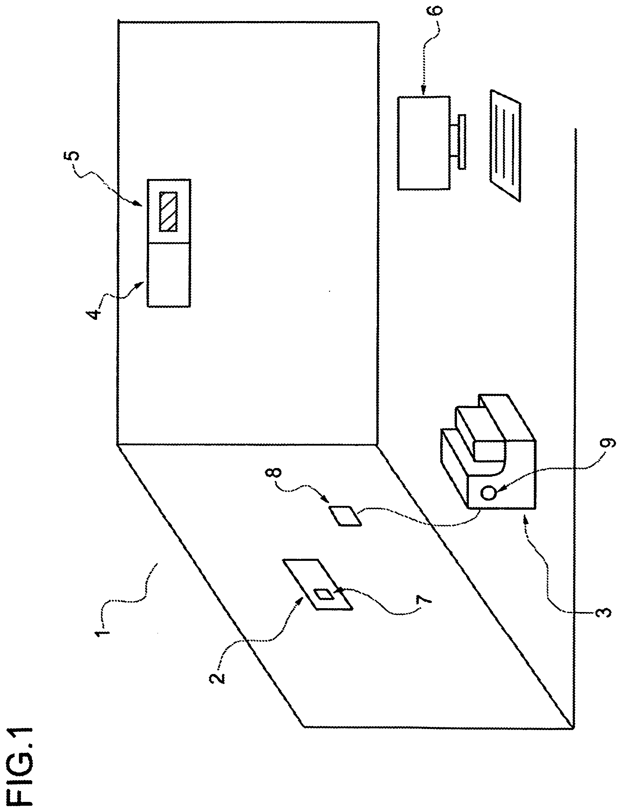

In Figure 1, the number 1 indicates a system for the execution, traceability, monitoring and control of a method of reducing the bacterial count according to the present invention.

This system 1 comprises an environment identification device 2, preferably fixed within the confined environment, and containing known storage means 7, containing the technical information relating to the method of reducing the bacterial count and the characteristics of the confined environment. In particular, the storage means 7 contain data representative of the periodicity of execution of the decontamination treatment.

The system 1 further comprises a micronizing device for micronizing air-dispersed decontaminant substances 3, comprising a transponder reader 8 for acquiring information about the confined environment to be treated (supplied by the environment identification device 2) and a diffuser 9 adapted to produce decontaminant substances in the form of dry fog.

The transponder 8 is also arranged to send to a central server 6 data relating to the method of reducing the bacterial count, such as the quantity of decontaminant substance delivered, the delivery time, and the like, said central server 6 coordinating the activity of the various devices of the system 1.

The data acquired by the central server 6 are processed in a known way by the server 6 itself so as to identify anomalies with respect to the expected objective and certify, by creating a "self-monitoring plan", the bacterial result obtained.

The processing of the data provides, in a known way, for the creation of graphs, the processing of operating protocols for prevention, the analysis of the costs of the process executed, and any other information useful for the improvement of the bacterial conditions of the confined environment, for the purpose of providing a virtual technical and scientific file on the confined environment.

The micronizing device 3 is capable of self-calibration on the basis of the information received from the environment identification device 2 and the data relating to the decontaminant substance, acquired for example by means of the transponder 8 arranged to read a code present on the packaging of the decontaminant substance.

Finally, the system 1 comprises a detection sensor 4 arranged to detect the concentration of the decontaminant substance delivered into the confined environment by the diffuser 9.

The system 1 further comprises an instantaneous bacterial analysis sensor 5 capable of determining the exact quantitative and qualitative bacterial level before and after the use of the method of reducing the bacterial count.

At the end of each cycle of bacterial count reduction, the micronizing device 3 operates in a known way to transfer the information on the executed activity to the central server 6. The micronizing device 3 also sends to the instantaneous bacterial analysis sensor 5 a signal to start the step of analysis of the qualitative and quantitative bacterial level present in the confined environment.

In turn, the instantaneous bacterial analysis sensor 5 sends the results of the completed analysis to the central server 6.

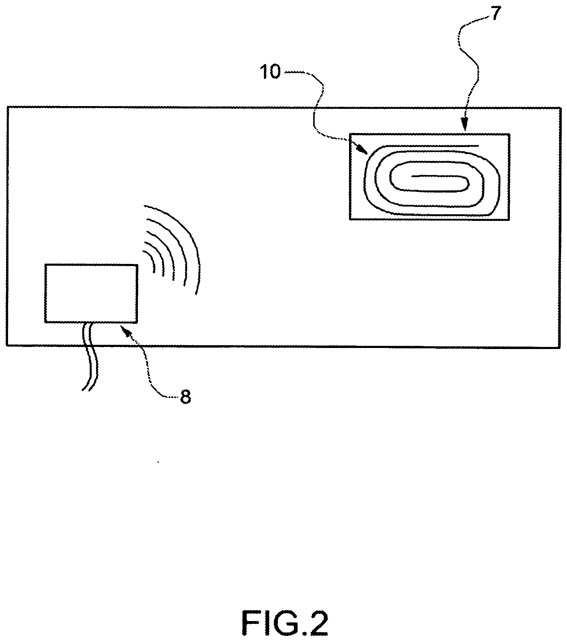

Figure 2, in which parts and elements identical or corresponding to those of Figure 1 have been given the same reference numerals, shows the environment identification device 2, comprising the memory means 7 containing a writable printed circuit 10 into which are input all the architectonic and volumetric information on the confined environment and the technical information relating to the method for reducing the bacterial count, such as the planned periodicity of treatment, thus determining the optimal concentration of product to be delivered.

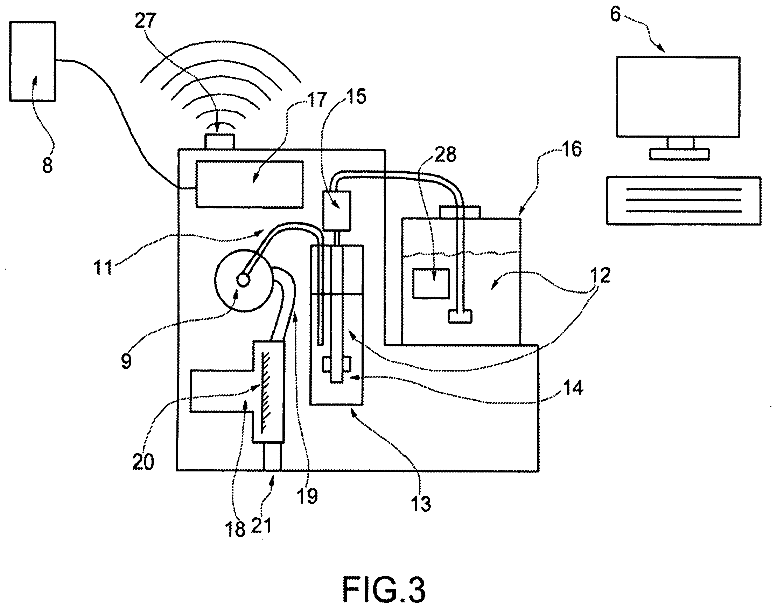

Figure 3, in which parts and elements identical or corresponding to those of Figure 1 have been given the same reference numerals, shows the micronizing device 3 in which the diffuser 9 diffuses decontaminant substances through the air and in the form of dry fog. The micronizing device 3 operates by the Venturi effect: a decontaminant substance 12 is drawn through a delivery tube 11 from a collecting reservoir 13, the level of which is kept constant by means of a float switch 14 connected to a squeeze pump 15 which fills the reservoir 13 by drawing the decontaminant substance 12 from the original retail pack 16. The decontaminant substance 12 is identified in respect of its organoleptic characteristics by means of a printed circuit positioned in a descriptive label 28 present on the pack 16.

The activity of all the components of the system according to the invention is controlled by a control unit 17 which, following the acquisition of the data on the confined environment by the transponder 8, starts the operation of an electric motor 18 connected to a blower 19 capable of proportionally mixing the decontaminant substance 12 with the quantity of air drawn in from the external environment by a fan 20 driven by the motor 18. The diffuser 9, operating by means of a data transmission system (not shown in the drawing), of the Bluetooth, SMS or GPRS type for example, transmits all the information about its own activity to the central server 6 for subsequent processing as described above.

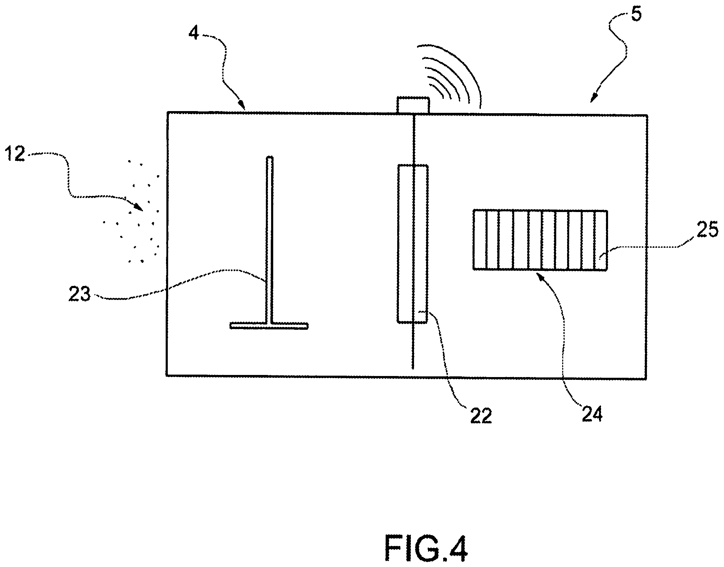

Figure 4, in which parts and elements identical or corresponding to those of Figure 1 have been given the same reference numerals, shows the detection sensor 4 for detecting the concentration of the decontaminant substance, comprising a piezoelectric probe 23 which is connected, through a microprocessor 22, to an instantaneous bacterial analysis sensor 5 based on a microfluidic platform of the lab-on-chip type 24 comprising sensitive microstructures 25 in the form of a "cantilever" or "array of cantilevers", arranged to capture any pathogenic microorganisms and to signal to the microprocessor 22 the quantity of microorganisms detected and their characteristics.

The microprocessor 22 is adapted to transfer the acquired information relating to the activity of the detection sensor 4 and the instantaneous bacterial analysis sensor 5, by means of a data transmission system of the Bluetooth, SMS or GPRS type 26 for example, to the central server 6 for subsequent processing as described above.

Advantageously, the system according to the present invention is capable, by means of the input into the central server 6 of qualitative and frequency parameters relating to activities included in the operational "self-monitoring plan" for a confined environment, of generating, on a preventive basis or following the discovery of contamination, reports of non-conformity of data and alarm signals which are transmitted in real time to the persons in charge of the operations and activities concerned.

Advantageously, the system according to the invention is capable of producing, by means of the systematic processing of the data obtained from the activity of the devices, statistical graphs and evaluation parameters for the costs compared with the direct and indirect benefits obtained in relation to the quality maintenance activity, while certifying the self-monitoring process specified in advance.

The method applied by the system according to the present invention makes it possible to achieve an optimal quality standard for a given confined environment, while avoiding procedures of in loco bacterial sampling by external operators and the consequent deterioration of bacterial quality found as a result of the previous decontamination process. For this purpose, it is no longer necessary to carry out operations of collecting and transporting sampled material in order to determine the bacterial level of the confined environment.

## Claims

1. System (1) for the execution, traceability, monitoring and control of a method of reducing the bacterial count in a confined environment, comprising:
- an environment identification device (2) containing information relating to the confined environment;
- a device (3) for micronizing a decontaminant substance (12) in the air in the form of a dry fog through a diffuser (9), based on the information contained in the environment identification device (2);
- a detection sensor (4) adapted to detect the concentration of the decontaminant substance (12); **characterised by**
- an instantaneous bacterial analysis sensor (5) arranged to determine the quantitative and qualitative bacterial concentration in the confined environment;
- a central server (6) arranged to process data received from the diffuser (9), from the detection sensor (4) and from the instantaneous bacterial analysis sensor (5), so as to identify anomalies with respect to a desired quantitative and qualitative bacterial objective and certify the bacterial result obtained as a result of the diffusion of the decontaminant substance (12).

2. System according to Claim 1, wherein the environment identification sensor (2) comprises memory means (7) containing technical information relating to the method for reducing the bacterial count in a confined environment.

3. System according to Claim 1 or 2, wherein the micronizing device (3) comprises:
- a collecting tank (13) adapted to contain the decontaminant substance (12);
- a tube (11) arranged to draw the decontaminant substance (12) from the collecting tank (13);
- a fan (20) arranged to draw air from the environment to send it to a blower (19) arranged to mix said air with the decontaminant substance (12) received from the collecting tank (13);
- a motor (18) adapted to drive the fan (20).

4. System according to Claim 3, wherein the collecting tank (13) contains a float switch (14) connected to a squeeze pump (15) arranged to fill the collecting tank (13) with the decontaminant substance (12) drawn from a retail pack (16) so as to maintain a predetermined level of the decontaminant substance (12) in the collecting tank (13).

5. System according to any one of the preceding claims, wherein the detection sensor (4) comprises a piezoelectric probe (23) connected, though a microprocessor (22), to the instantaneous bacterial analysis sensor (5).

6. System according to any of the preceding claims, wherein the instantaneous bacterial analysis sensor (5) is based on a microfluidic platform of the lab-on-chip type (24) comprising sensitive microstructures (25) in the form of a "cantilever" or "array of cantilevers", arranged to capture any pathogenic microorganisms and to signal to the microprocessor (22) the quantity of microorganisms detected and their characteristics.

## Patentansprüche

1. System (1) zur Durchführung, Rückverfolgung, Überwachung und Steuerung eines Verfahrens zur Verminderung der Bakterienanzahl in einer begrenzten Umgebung, umfassend:
- eine Umgebungsidentifizierungsvorrichtung (2), die Informationen in Bezug auf die begrenzte Umgebung enthält,
- eine Vorrichtung (3) zum Mikronisieren einer Dekontaminierungssubstanz (12) in der Luft in der Form eines trockenen Nebels durch einen Diffusor (9) auf der Basis der Informationen, die in der Umgebungsidentifizierungsvorrichtung (2) enthalten sind,
- einen Erfassungssensor (4), der zum Erfassen der Konzentration der Dekontaminierungssubstanz (12) angepasst ist,
**gekennzeichnet durch**
- einen schnell ansprechenden Sensor für eine bakterielle Analyse (5), der zur quantitativen und qualitativen Bestimmung der bakteriellen Konzentration in der begrenzten Umgebung angeordnet ist,
- einen zentralen Server (6), der zum Verarbeiten von Daten, die von dem Diffusor (9), von dem Erfassungssensor (4) und von dem schnell ansprechenden Sensor für eine bakterielle Analyse (5) erhalten werden, angeordnet ist, so dass Anomalien in Bezug auf ein gewünschtes quantitatives und qualitatives bakterielles Ziel identifiziert werden und das bakterielle Ergebnis, das als Ergebnis der Diffusion der Dekontaminierungssubstanz (12) erhalten worden ist, bestätigt wird.

2. System nach Anspruch 1, bei dem der Umgebungsidentifizierungssensor (2) Speichermittel (7) umfasst, die technische Informationen in Bezug auf das Verfahren zum Vermindern der Bakterienanzahl in einer begrenzten Umgebung enthalten.

3. System nach Anspruch 1 oder 2, bei dem die Mikronisierungsvorrichtung (3) umfasst:
- einen Vorratsbehälter (13), der zum Aufnehmen der Dekontaminierungssubstanz (12) angepasst ist,
- einen Schlauch oder ein Rohr (11), der oder das zum Entnehmen der Dekontaminierungssubstanz (12) aus dem Vorratsbehälter (13) angeordnet ist,
- ein Lüfterrad (20), das zum Ziehen von Luft aus der Umgebung und Leiten der Luft zu einem Gebläse (19) angeordnet ist, das zum Mischen der Luft mit der Dekontaminierungssubstanz (12) angeordnet ist, die von dem Vorratsbehälter (13) erhalten worden ist,
- einen Motor (18), der zum Antreiben des Lüfterrads (20) angepasst ist.

4. System nach Anspruch 3, bei dem der Vorratsbehälter (13) einen Schwimmerschalter (14) enthält, der mit einer Schlauchpumpe (15) verbunden ist, die zum Füllen des Vorratsbehälters (13) mit der Dekontaminierungssubstanz (12) angeordnet ist, die von einer Verkaufspackung (16) entnommen wird, so dass ein vorgegebenes Niveau der Dekontaminierungssubstanz (12) in dem Vorratsbehälter (13) aufrechterhalten wird.

5. System nach einem der vorhergehenden Ansprüche, bei dem der Erfassungssensor (4) eine piezoelektrische Sonde (23) umfasst, die durch einen Mikroprozessor (22) mit dem schnell ansprechenden Sensor für eine bakterielle Analyse (5) verbunden ist.

6. System nach einem der vorhergehenden Ansprüche, bei dem der schnell ansprechende Sensor für eine bakterielle Analyse (5) auf einer Mikrofluidplattform des Chiplabor ("lab-on-chip")-Typs (24) basiert, die erfassende Mikrostrukturen (25) in der Form eines "Auslegers" ("cantilever") oder einer "Gruppierung von Auslegern" umfasst, und die so angeordnet ist, dass sie jedwede pathogenen Mikroorganismen erfasst und die Menge der erfassten Mikroorganismen und deren Eigenschaften an den Mikroprozessor (22) weitergibt.

## Revendications

1. Système (1) pour l'exécution, la traçabilité, la surveillance et le contrôle d'un procédé de réduction du nombre de bactéries dans un environnement confiné, comprenant :
- un dispositif d'identification de l'environnement (2) contenant des informations liées à l'environnement confiné ;
- un dispositif (3) de micronisation d'une substance décontaminante (12) dans l'air sous la forme d'un brouillard sec par l'intermédiaire d'un diffuseur (9), basé sur les informations contenues dans le dispositif d'identification de l'environnement (2) ;
- un capteur de détection (4) conçu pour détecter la concentration de la substance décontaminante (12) ; **caractérisé par**
- un capteur d'analyse bactérienne instantanée (5) conçu pour déterminer la concentration bactérienne quantitative et qualitative dans l'environnement confiné ;
- un serveur central (6) conçu pour traiter les données provenant du diffuseur (9), du capteur de détection (4) et du capteur d'analyse bactérienne instantanée (5), de manière à identifier des anomalies par rapport à un objectif bactérien quantitatif et qualitatif recherché et confirmer le résultat bactérien obtenu suite à la diffusion de la substance décontaminante (12).

2. Système selon la revendication 1, dans lequel le capteur d'identification de l'environnement (2) comprend un moyen de mémoire (7) contenant des informations techniques liées au procédé de réduction du nombre de bactéries dans un environnement confiné.

3. Système selon la revendication 1 ou 2, dans lequel le dispositif de micronisation (3) comprend :
- un réservoir de collecte (13) conçu pour contenir la substance décontaminante (12) ;
- un tube (11) conçu pour prélever la substance décontaminante (12) du réservoir de collecte (13) ;
- un ventilateur (20) conçu pour aspirer l'air de l'environnement et l'envoyer vers un souffleur (19) conçu pour mélanger ledit air avec la substance décontaminante (12) provenant du réservoir de collecte (13) ;
- un moteur (18) conçu pour entraîner le ventilateur (20).

4. Système selon la revendication 3, dans lequel le réservoir de collecte (13) contient un contacteur à flotteur (14) relié à une pompe péristaltique (15) conçue pour remplir le réservoir de collecte (13) de la substance décontaminante (12) prélevée dans un produit de vente au détail (16) de manière à maintenir un niveau prédéterminé de la substance décontaminante (12) dans le réservoir de collecte (13).

5. Système selon l'une quelconque des revendications précédentes, dans lequel le capteur de détection (4) comprend une sonde piézoélectrique (23) connectée, par un microprocesseur (22), au capteur d'analyse bactérienne instantanée (5).

6. Système selon l'une quelconque des revendications précédentes, dans lequel le capteur d'analyse bactérienne instantanée (5) est basé sur une plateforme microfluidique du type laboratoire-sur-puce (24) comprenant des microstructures sensibles (25) sous la forme d'un « support » ou « réseau de supports », conçues pour capturer les micro-organismes pathogènes et signaler au microprocesseur (22) la quantité de micro-organismes détectés et leurs caractéristiques.
